# EUROPEAN PATENT SPECIFICATION

(11) **EP 0 755 927 B1**
(45) Date of publication and mention of the grant of the patent: **18.07.2001**
(21) Application number: 94912688.2
(22) Date of filing: 15.04.1994
(51) Int. Cl.: C07D 307/68, B27K 3/34

(54) **DIMETHYLFURANCARBOXANILIDE DERIVATIVE**
DIMETHYLFURANCARBOXAMILID-DERIVAT
DERIVE DE DIMETHYLFURANCARBOXANILIDE

(43) Date of publication of application: 29.01.1997
(73) Proprietor: SANKYO COMPANY LIMITED, Chuo-ku, Tokyo 103-0023 (JP)
(72) Inventor: KONISHI, Kiyoshi Sankyo Company, Limited, Yasu-cho, Yasu-gun Shiga-ken 520-23 (JP); YANAI, Toshiaki Sankyo Company, Limited, Yasu-cho, Yasu-gun Shiga-ken 520-23 (JP); SAITO, Akio Sankyo Company Limited, Shinagawa-ku Tokyo 140 (JP)
(74) Representative: Gibson, Christian John Robert
(86) International application number: JP9400631
(87) International publication number: WO9510511

(56) References cited:
- EP-A- 0 589 313
- EP-A- 0 597 336
- JP-A- 5 221 994
- CHEMICAL ABSTRACTS, vol. 119, no. 11, 13 September 1993 Columbus, Ohio, US; abstract no. 111279, KONISHI SEIJI ET AL.: "Synergistic wood preservatives containing 2,5-dimethylfuran-3- carboxanilide" XP002029667 & JP 05 112 406 A (SANKYO CO.)

## Description

### [Technological field]

The present invention is concerned with novel dimethylfurancarboxyanilide derivatives exhibiting an excellent antimicrobial effect, a wood preservative containing the dimethylfurancarboxyanilide derivative as the active ingredient, and a wood preservative composition in which the dimethylfurancarboxyanilide derivative as one of the active ingredients is combined with any commercially available wood preservative the effect of which has been already confirmed.

### [Background technology]

Various kinds of inorganic or organic compounds have previously been employed to preserve timber against decay due to various wood-rotting fungi. However, these chemicals have faults such as affecting the human body because of their high toxicity, showing environmental polution, requiring a high concentration thereof when employed and being expensive.

As for compounds relating to the dimethylfurancarboxyanilide derivatives of the present invention, compounds represented by the formula below have been disclosed in Japanese Patent Kokai Application Sho 50-10376 as a chemical for preventing plant injury; in which, however, R is limited to phenyl, nitro-substituted phenyl, carboxy-substituted phenyl, phenyl-substituted phenyl, methyl-substituted phenyl, halogen-substituted phenyl or methoxy-substituted phenyl. In addition, this patent is silent on the other derivatives, and no activity of these compounds on wood-rotting fungi has been described.

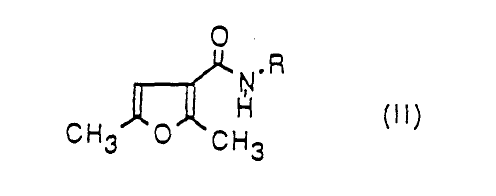

JP-A-5112406 discloses the compound 2,5-dimethylfurancarboxyanilide which is said to have wood preservative properties. GB 1,592,837 discloses N-halogenalkylthio derivatives of 2,5-dimethylfurancarboxyanilide compounds which are said to have wood preservative activity and intermediates for their production; some of these intermediates are shown in the present application to have wood preservative properties.

### [Process and constitution of Invention]

The object of the present invention exists in providing a novel wood preservative which is safer, and is possible to use effectively at a low concentration and/or at a low price.

In consideration of such situation as mentioned above, the present inventors considered furancarboxyanilide derivatives, and studied eagerly. Our study resulted in finding that novel dimethylfurancarboxyanilide derivatives represented by the general formula (I) below are very useful as a wood preservative and furthermore, if the dimethylfurancarboxyanilide derivative as the active ingredient is combined with any other commercially available wood preservative, potentiation effect can be observed and a wood preservative composition can be prepared.

The compounds of the present invention are the dimethylfurancarboxyanilide derivatives represented by the general formula 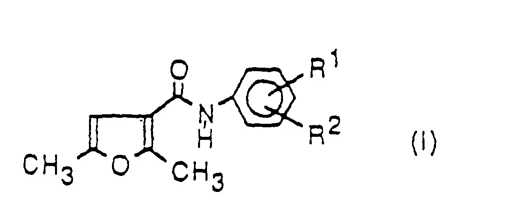 wherein
R¹ represents:
a 3-alkoxycarbonyl group having from 1 to 3 carbon atoms in the alkoxy moiety;
a 3-alkoxymethyl group having from 1 to 3 carbon atoms in the alkoxy moiety;
a cycloalkylcarbonylamino group having from 4 to 6 carbon atoms in the cycloalkyl moiety;
a benzyl group which may be substituted by a methoxy group;
a benzoyl group; or
an alkoxycarbonylalkenyl group having from 1 to 3 carbon atoms in the alkoxy moiety and having from 2 to 3 carbon atoms in the alkenyl moiety; and
R² represents a hydrogen atom.

In a second aspect, the present invention also provides a wood preservative composition comprising a carrier in combination with an amount effective in preserving wood of dimethylfurancarboxyanilide derivative of general formula (I') as defined below as the active ingredient: 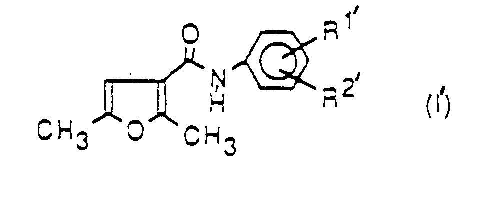 wherein: R^{1'} represents:
a 3-alkyl group having from 2 to 6 carbon atoms;
a 3-alkoxycarbonyl group having from 1 to 3 carbon atoms in the alkoxy moiety;
a 3-alkoxymethyl group having from 1 to 3 carbon atoms in the alkoxy moiety;
a cycloalkylcarbonylamino group having from 4 to 6 carbon atoms in the cycloalkyl moiety;
a benzyl group which may be substituted by a methoxy group;
a benzoyl group; or
an alkoxycarbonylalkenyl group having from 1 to 3 carbon atoms in the alkoxy moiety and having from 2 to 3 carbon atoms in the alkenyl moiety; and
R²' represents a hydrogen atom.

In a third aspect, there is also provided a method of preserving wood comprising applying to the wood a composition comprising an amount effective in the treatment of wood of a dimethylfurancarboxyanilide derivative of general formula (I') as defined above.

In a fourth aspect, there is yet further provided a wood preservative composition wherein at least one dimethylfurancarboxyanilide derivative of general formula (I') as defined above is combined with at least one compound selected from 3-bromo-2,3-diiodo-2-propenylethylcarbamate, 3-iodo-2-propynylbutylcarbamate and 4-chlorophenyl-3-iodopropargylformal.

### [The best mode for working of the invention]

In the general formula (I) above, as an alkyl group having from 2 to 6 carbon atoms, which is included in the definitions for R¹, there may be mentioned a straight or branched chain alkyl group such as ethyl, propyl, isopropyl, butyl, isobutyl, sec-butyl, tert-butyl, pentyl, isopentyl, neo-pentyl, hexyl, isohexyl or sec-hexyl; particularly preferably an alkyl group having from 2 to 6 carbon atoms.

In the general formula (I) above, as an alkoxy group having from 1 to 3 carbon atoms contained in an alkoxymethyl group having from 1 to 3 carbon atoms in the alkoxy moiety, which is included in the definitions for R¹, there may be mentioned a straight or branched chain alkoxy group such as methoxy, ethoxy, propoxy and isopropoxy.

In the general formula (I) above, as an alkoxycarbonyl group having from 1 to 3 carbon atoms in the alkoxy moiety, which is included in the definitions for R¹, there may be mentioned a group which is formed from the aforementioned alkoxy group having from 1 to 3 carbon atoms contained in an alkoxymethyl group having from 1 to 3 carbon atoms in the alkoxy moiety and from a carbonyl group, such as methoxycarbonyl, ethoxycarbonyl and isopropoxycarbonyl.

In the general formula (I) above, as a cycloalkylcarbonylamino group having from 4 to 6 carbon atoms in the cycloalkyl moiety, which is included in the definitions for R¹, there may be mentioned cyclobutylcarbonylamino, cyclopentylcarbonylamino or cyclohexylcarbonylamino; preferably cyclohexylcarbonylamino.

In the general formula (I) above, as a benzyl group which may have optionally a methoxy substituent, a benzyl group is preferred.

In the general formula (I) above, as an alkoxycarbonylalkenyl group having from 1 to 3 carbon atoms in the alkoxy moiety and having from 2 to 3 carbon atoms in the alkenyl moiety, which is included in the definitions for R¹, there may be mentioned methoxycarbonylvinylene, or ethoxycarbonyl-2-propenylene; preferably methoxycarbonylvinylene.

Novel dimethylfurancarboxyanilide derivatives which may be used as an active ingredient of the wood preservative of the present invention are exemplified in the following table.

In Table 1 below, abbreviations are used as follows.

| | |
|---|---|
| Bz | Benzyl |
| Et | Ethyl |
| Hx | Hexyl |
| Me | Methyl |
| Ph | Phenyl |
| Pn | Pentyl |
| Pr | Propyl |
| i | iso |
| c | cyclo |

**Table 1**

| Compound No. | R¹ | R² |
|---|---|---|
| 3 | 3-CH₂OMe | H |
| 10 | 3-CH₂OEt | H |
| 26 | 3-CH₂OPr | H |
| 27 | 3-CH₂OiPr | H |
| 69 | 3-COOMe | H |
| 70 | 3-COOEt | H |
| 71 | 3-COOPr | H |
| 72 | 3-COOiPr | H |
| 75 | 3-COPh | H |
| 80 | 3-NHCOcPn | H |
| 81 | 3-NHCOcHx | H |
| 82 | 3-Bz | H |
| 83 | 3-(4-MeOBz) | H |
| 85 | 3-CH=CHCOOMe | H |

Among the compounds above, preferred ones include Compound Nos. 3, 10, 26, 27, 69, 70, 71, 72, 75, 80, 81, 82, 83 and 85; and more preferred ones include Compound Nos. 3, 10, 26, 27, 69, 70, 81, 83 and 85.

The compounds of the said general formula (I) may be prepared according to the procedure summarized in either the following Method A or Method B.

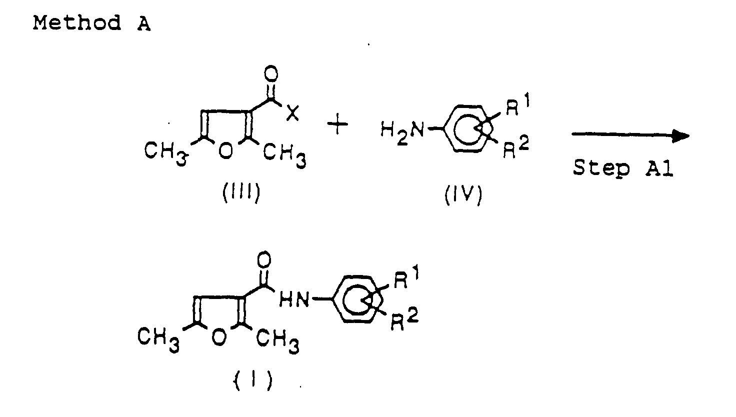

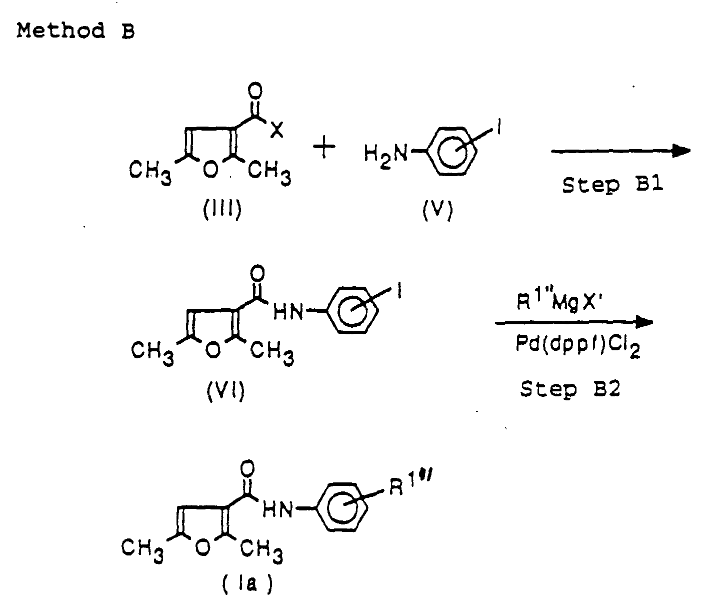

In the above formulae, R¹ and R² are as defined above. R^{1''} represents a C₂-C₆ alkyl group or a benzyl group which may optionally have a methoxy substituent. The compound of formula (Ia) is that of a general formula (I'), in which R^{1'} is R^{1''} and R² signifies a hydrogen atom. The compound of formula (V) is iodine-substituted aniline. X signifies a halogen atom such as chlorine, bromine or iodine, preferably chlorine. X' signifies a halogen atom such as a chlorine, bromine or iodine, preferably bromine or iodine.

The compounds of the present invention may be prepared by well-known procedure.

Step A1 consists of the preparation of a compound of general formula (I) by reacting a compound of general formula (III) with a compound of general formula (IV) in an inert solvent in the presence of a dehydrohalogenating agent.

A compound of formula (III) used as a starting material in this step may be prepared by hydrolyzing 2,5-dimethylfuran-3-carboxylate, which may be prepared by condensing chloroacetone with acetoacetate, followed by halogenation.

A compound of formula (IV) used as a starting material in this step is an aniline derivative which is commercially available or may be prepared by well-known methods.

Examples of the inert solvents used include, for example, ethers such as ether, isopropyl ether, tetrahydrofuran or dioxane; aromatic hydrocarbons such as benzene, toluene or xylene; halogenated hydrocarbons such as dichloromethane, chloroform or carbon tetrachloride; and mixtures of two or more of these solvents; preferably aromatic hydrocarbons (particularly toluene).

Examples of dehydrohalogenating agents used include, for example, tertiary amines such as triethylamine, N,N-dimethylaminopyridine or the like and pyridines. This reaction can be carried out in the presence or absence of a solvent. In order to perform the reaction smoothly, using a solvent, the reaction is carried out at a temperature of 0°C to reflux temperature of the solvent used, preferably from room temperature to 100°C. The time required for the reaction takes generally from 30 minutes to 5 hours, preferably from 30 minutes to 2 hours.

Step B1 consists of the preparation of a compound having general formula (VI) by reacting a compound having general formula (III) with a compound having general formula (V) in an inert solvent in the presence of a dehydrohalogenating agent.

A compound of formula (IV) used as a starting material in this step is an aniline derivative which is commercially available or may be prepared by well-known methods.

The reaction conditions employed in this step are similar to those employed in Step A1.

Step B2 consists of the preparation of a compound having general formula (Ia) by reacting a compound having general formula (VI) with a Grignard reagent having general formula:
R¹''MgX' in an inert solvent in the presence of a catalyst.

Examples of preferred inert solvents used include, for example, ethers such as diethyl ether, isopropyl ether, tetrahydrofuran or dioxane; particularly preferably diethyl ether.

As a particularly preferred catalyst there may be used [1,1'-bis(diphenylphosphino)ferrocene]palladium(II) chloride.

The Grignard reagents used in this process are commercially available or can be prepared by reacting magnesium with an alkyl halide represented by formula:
R¹''X' (wherein R¹'' and X' are as defined above) according to well-known methods.

The reaction is normally carried out at a temperature of 0°C to 50°C, preferably at room temperature. Although the time required for the reaction varies depending upon the nature of the solvent and reagent to be used, the reaction is normally complete within a period of 10 hours to 10 days.

The compounds having the said general formula (I) in according with the invention have potent wood preservative activity at low concentration, compared with the activity shown by existing wood preservatives. A composition consisting of a combination of the foregoing compound (I) with a known wood preservative gives a synergistic effect, lower concentrations of each compound being required than would be expected from the activity shown by each singly, such that the composition shows efficient wood preservative activity at a low concentration. Therefore, novel dimethylfurancarboxyanilide derivatives are extremely effective as a wood preservative in low concentration so as to solve one of the problems in the quality of life.

The following Examples illustrate the preparation and the formulation of the compound of the invention in more detail. Such examples are not to be construed as being limitative of the scope of the invention.

### Example 1

### 3'-Acetylamino-2,5-dimethylfuran-3-carboxanilide

To a solution of 0.50 g of 2,5-dimethylfuran-3-carbonyl chloride in 10 ml of dichloromethane were added 0.44 ml of triethylamine and 0.47 g of 3-acetylaminoaniline under ice-cooling, and the resulting mixture was stirred at room temperature for 2.5 hours followed by heating under reflux for 4.5 hours. After the reaction mixture was cooled, it was diluted by adding 10 ml of dichloromethane. The diluted mixture was successively washed with 1 N sodium hydroxide, 1 N hydrochloric acid and a saturated aqueous solution of sodium chloride and dried over sodium sulfate followed by distilling off the solvent. The residue was purified by column chromatography through silica gel and the desired fractions were recrystallized from ethyl acetate to give 0.51 g of the desired compound as white crystals in a 59.4% yield.
m.p.: 172.0-172.5°C
¹H NMR (CDCl₃ + DMSO) δ ppm:
8.4 (1H, b), 7.95 (1H, b), 7.88 (1H, m), 7.4 (1H, m), 7.32 (1H, m), 7.25 (1H, t, J=8Hz), 6.25 (1H, s), 2.55 (3H, s), 2.25 (3H, s), 2.15 (3H, s)
IR (KBr) cm⁻¹: 3306, 1672, 1651, 1086, 781
Elemental analysis (%): Calc'd for C₁₅H₁₆N₂O₃: C, 66.16; H, 5.92; N, 10.29. Found: C, 66.30; H, 5.98; N, 10.32.

Following the similar procedure as above, which produces a compound which is not a part of the present invention, but using an appropriate aniline derivative instead of 3-acetylaminoaniline, there were obtained the following compounds of the present invention.

### Example 6

### 3'-Methoxycarbonyl-2,5-dimethylfuran-3-carboxanilide

Yield: 77.1%
m.p.: 104.0-106.0°C
¹H NMR (CDCl₃) δ ppm:
8.05 (1H, m), 7.98 (1H, m), 7.8 (1H, m), 7.42 (1H, t, J=8Hz), 7.38 (1H, b), 6.1 (1H, s), 3.92 (3H, s), 2.55 (3H, s), 2.25 (3H, s)
IR (KBr) cm⁻¹: 3437, 1704, 1675, 1070, 759
Elemental analysis (%): Calc'd for C₁₅H₁₅NO₄: C, 65.92; H, 5.53; N, 5.13. Found: C, 66.02; H, 5.60; N, 5.08.

### Example 7

### 3'-Benzoyl-2,5-dimethylfuran-3-carboxanilide

Yield: 69.1%
m.p.: 137.0-139.0°C
¹H NMR (CDCl₃) δ ppm:
8.05 (1H, m), 7.85-7.7 (3H, m), 7.6 (1H, m), 7.55-7.35 (5H, m), 6.1 (1H, s), 2.55 (3H, s), 2.25 (3H, s)
IR (KBr) cm⁻¹: 3386, 1672, 1647, 1069, 707
Elemental analysis (%): Calc'd for C₂₀H₁₇NO₃: C, 75.22; H, 5.37; N, 4.39. Found: C, 75.38; H, 5.43; N, 4.38.

### Example 10

### 3'-Cyclohexylcarbonylamino-2,5-dimethylfuran-3-carboxanilide

Yield: 45.1%
m.p.: 212.5-213.0°C
¹H NMR (CDCl₃) δ ppm:
7.92 (1H, b), 7.88 (1H, b), 7.45-7.35 (2H, m), 7.25 (1H, t, J=8Hz), 6.22 (1H, s), 2.55 (3H, s), 2.25 (3H, s), 2.25-2.2 (1H, m), 2.0-1.2 (10H, m)
IR (KBr) cm⁻¹: 3238, 1651, 1639, 1076, 781
Elemental analysis (%): Calc'd for C₂₀H₂₄N₂O₃: C, 70.57; H, 7.11; N, 8.23. Found:C, 70.56; H, 7.26; N, 8.16

### Example 11

### 3'-Methoxymethyl-2,5-dimethylfuran-3-carboxanilide

Yield: 73.3%
m.p.: 102.5-103.5°C
¹H NMR (CDCl₃) δ ppm:
7.55 (1H, m), 7.52 (1H, d, J=8Hz), 7.32 (1H, t, J=8Hz), 7.32 (1H, b), 6.9 (1H, d, J=8Hz), 6.1 (1H, s), 4.45 (2H, s), 3.4 (3H, s), 2.55 (3H, s), 2.25 (3H, s)
IR (KBr) cm⁻¹: 3278, 1645, 1237, 1107, 784
Elemental analysis (%): Calc'd for C₁₅H₁₇NO₃: C, 69.48; H, 6.61; N, 5.40. Found: C, 69.22; H, 7.02; N, 5.37.

### Example 12

### 3'-Ethoxymethyl-2,5-dimethylfuran-3-carboxanilide

Yield: 64.4%
m.p.: 85.0-85.5°C.
¹H NMR (CDCl₃) δ ppm:
7.65-7.55 (2H, m), 7.38 (1H, t, J=8Hz), 7.35 (1H, b), 7.15 (1H, d, J=8Hz), 6.15 (1H, s), 4.55 (2H, s), 3.58 (2H, q, J=8Hz), 2.55 (3H, s), 2.25 (3H, s), 1.3 (3H, t, J=8Hz)
IR (KBr) cm⁻¹: 3279, 1646, 1115, 785
Elemental analysis (%): Calc'd for C₁₆H₁₉NO₃: C, 70.31; H,
7.01; N, 5.12. Found: C, 70.14; H, 7.27; N, 5.06.

### Example 13

### 3'-Isopropyloxymethyl-2,5-dimethylfuran-3-carboxanilide

Yield: 92.7%
m.p.: 68.0-69.5°C
¹H NMR (CDCl₃) δ ppm:
7.55 (1H, d, J=8Hz), 7.5 (1H, m), 7.3 (1H, t, J=8Hz), 7.3 (1H, b), 7.12 (1H, d, J=8Hz), 6.1 (1H, s), 4.5 (2H, s), 3.7 (1H, m), 2.55 (3H, s), 2.25 (3H, s), 1.25 (6H, d, J=7Hz)
IR (Liquid film) cm⁻¹: 3321, 1651, 1072, 785
Elemental analysis (%): Calc'd for C₁₇H₂₁NO₃: C, 71.06; H,
7.37; N, 4.87. Found: C, 70.35; H, 7.14; N, 4.91.

### Example 14

### 3'-(4-Methoxybenzyl)-2,5-dimethylfuran-3-carboxanilide

Yield: 86.8%
m.p.: 100.0-102.5°C
¹H NMR (CDCl₃) δ ppm:
7.45 (1H, m), 7.35 (1H, m), 7.25 (1H, t, J=8Hz), 7.25 (1H, b), 7.1 (2H, d, J=8Hz), 6.92 (1H, d, J=8Hz), 6.88-6.75 (1H, m), 6.82 (2H, d, J=8Hz), 6.05 (1H, s), 3.9 (2H, s), 3.75 (3H, s), 2.55 (3H, s), 2.25 (3H, s)
IR (KBr) cm⁻¹: 3345, 1656, 1246, 1074, 694
Elemental analysis (%): Calc'd for C₂₁H₂₁NO₃: C, 75.20; H,
6.31; N, 4.18. Found: C, 75.28; H, 6.32; N, 4.21.

### Example 15

### 3'-(2-Methoxycarbonylvinyl)-2,5-dimethylfuran-3-carboxanilide

Yield: 63.3%
m.p.: 159.5-161.5°C
¹H NMR (CDCl₃) δ ppm:
7.82 (1H, m), 7.7 (1H, d, J=15Hz), 7.58 (1H, m), 7.38 (1H, b), 7.35 (1H, t, J=8Hz), 7.28 (1H, m), 6.48 (1H, d, J=15Hz), 6.12 (1H, s), 3.82 (3H, s), 2.55 (3H, s), 2.25 (3H, s)
IR (KBr) cm⁻¹: 3387, 1685, 1670, 1068, 800
Elemental analysis (%): Calc'd for C₁₇H₁₇NO₄: C, 68.22; H, 5.72; N, 4.68. Found: C, 67.55; H, 5.64; N, 4.62.

### Example 29

### 3'-Benzyl-2,5-dimethylfuran-3-carboxanilide

Yield: 59.8%
m.p.: 123.0-125.0°C
¹H NMR (CDCl₃) δ ppm:
7.45 (1H, m), 7.38 (1H, m), 7.35-7.15 (7H, m), 6.95 (1H, d, J=8Hz), 3.98 (2H, s), 2.55 (3H, s), 2.25 (3H, s)
IR (KBr) cm⁻¹: 3314, 1640, 1078, 777, 701
Elemental analysis (%): Calc'd for C₂₁H₁₉NO₂: C, 78.66; H, 6.27; N, 4.59. Found: C, 77.76; H, 6.28; N, 4.55

### Referential Example 1

### Ethyl 2,5-dimethylfuran-3-carboxylate

To a suspension of 2.4 g of sodium hydride (60% dispersion in mineral oil) in 10 ml of N,N-dimethylformamide (hereinafter, abbreviated as DMF) a solution of 6.5 ml of ethyl acetoacetate in 5 ml of DMF was added dropwise with stirring under ice-cooling, and 5.97 ml of chloroacetone were added dropwise thereto with stirring under ice-cooling. After stirring at room temperature for 3 hours, the reaction mixture was poured into water and the aqueous mixture was extracted with ethyl acetate. The extract was washed with a saturated aqueous solution of sodium chloride and dried over anhydrous sodium sulfate. After distilling off the solvent under reduced pressure, the residue was distilled in vacuo to give 8.01 g of ethyl-α-acetonylacetoacetate having b.p. 105°C/2 mmHg in a 86% yield.

To a solution of the ester thus obtained in 20 ml of ethanol were added 2 g of p-toluenesulfonic acid, and the resulting mixture was heated under reflux for 2 hours. The reaction mixture was allowed to cool to room temperature and the solvent was distilled off under reduced pressure. The residue was dissolved in ethyl acetate and the solution was washed with a saturated aqueous solution of sodium chloride followed by drying over anhydrous magnesium sulfate. After distilling off the solvent under reduced pressure, the residue was purified by column chromatography through silica gel using a 10:1 mixture of n-hexane and ethyl acetate as an eluent to give 5.14 g of ethyl 2,5-dimethylfuran-3-carboxylate in a 71% yield.

### Referential Example 2

### 2,5-Dimethylfuran-3-carboxylic acid

A mixture of 3.2 g of ethyl 2,5-dimethylfuran-3-carboxylate, 35 ml of ethanol and 20 ml of 2 N sodium hydroxide was stirred at room temperature for 1.5 hours followed by heating under reflux for an hour. After the reaction mixture was allowed to cool to room temperature, it was concentrated under reduced pressure. The residue was dissolved in water and acidified with diluted sulfuric acid.

Precipitated crystals were collected by filtration, washed with water and dried to give 2.27 g of 2,5-dimethylfuran-3-carboxylic acid in a 85% yield.

The following compounds of Referential Examples 3 and 4 were prepared in the same manner as the compound of Example 1.

### Referential Example 3

### 3'-Ethyl-2,5-dimethylfuran-3-carboxanilide

Yield: 91.0%
m.p.: 113-115°C
Mass (m/z): 243 (M⁺), 123.94
¹H NMR (CDCl₃) δ ppm:
7.47-6.95 (4H, m), 6.1 (1H, s), 2.66 (3H, q), 2.60 (3H, s), 2.29 (3H, s) , 1.25 (3H, t)

### Referential Example 4

### 3'-Isopropyl-2,5-dimethylfuran-3-carboxanilide

Yield: 84.0%
m.p.: 79-80°C
Mass (m/z): 257 (M⁺), 149.135
¹H NMR (CDCl₃) δ ppm:
7.47-6.98 (4H, m), 6.11 (1H, s), 2.91 (1H, q,q), 2.60 (3H, s), 2.29 (3H, s), 1.26 (d, 6H)

### Referential Example 5

### 3'-Hexyl-2,5-dimethylfuran-3-carboxanilide

(Step 1) To a solution of 3.95 g of 2,5-dimethylfuran-3-carbonyl chloride in 60 ml of dichloromethane were added 3.45 ml of triethylamine and 2.99 ml of m-iodoaniline under ice-cooling, and the resulting mixture was stirred at room temperature for 6.5 hours. After the reaction mixture was cooled, it was diluted by adding 50 ml of dichloromethane. The diluted mixture was successively washed with 1 N sodium hydroxide, 1 N hydrochloric acid and a saturated aqueous solution of sodium chloride and dried over sodium sulfate followed by distilling off the solvent. The residue was subjected to column chromatography through silica gel to give 7.64 g of 2,5-dimethylfuran-3-carboxy(3-iodoanilide) as pale-yellow crystals in a 89.9% yield.

(Step 2) To a solution of 0.68 g of the crystals obtained in Step 1 in 8 ml of diethyl ether were added 29.3 mg of [1,1'-bis(diphenylphosphino)ferrocene]palladium(II) chloride and 11 ml of 1 M hexylmagnesium bromide, prepared from hexyl bromide and magnesium, divided into six equal parts, and the resulting mixture was stirred at room temperature for 47 hours. After adding 2 N hydrochloric acid to the reaction mixture, the catalyst was filtered off and the filtrate was extracted with diethyl ether. The extract was successively washed with an aqueous solution of sodium bicarbonate and a saturated aqueous solution of sodium chloride and dried over sodium sulfate. After distilling off the solvent, the residue was purified by chromatography through silica gel and then D-ODS-5, YMC-packed column to give 316 mg of the desired compound as white crystals in a 52.8% yield.
m.p.: 71.5-72.0°C
¹H NMR (CDCl₃) δ ppm:
7.45 (1H, m), 7.35 (1H, m), 7.25 (1H, b), 7.22 (1H, t, J=8Hz), 6.95 (1H, d, J=8Hz), 6.1 (1H, s), 2.65-2.5 (2H, m), 2.55 (3H, s), 2.25 (3H, s), 1.7-1.5 (2H, m), 1.4-1.2 (6H, m), 0.85 (3H, t, J=7Hz)
IR (KBr) cm⁻¹: 3310, 1643, 1077, 788
Elemental analysis (%): Calc'd for C₁₉H₂₅NO₂: C, 76.22; H, 8.42; N, 4.68. Found: C, 76.15; H, 8.54; N, 4.55

Following the similar procedure as above, but using an appropriate Grignard reagent instead of hexylmagnesium bromide, there were obtained the following compounds.

### Referential Example 6

### 3'-Butyl-2,5-dimethylfuran-3-carboxanilide

Yield: 36.4%
m.p.: 77.0-80.0°C
¹H NMR (CDCl₃) δ ppm: 7.45 (1H, m), 7.35 (1H, m), 7.25 (1H, b), 7.22 (1H, t, J=8Hz), 6.95 (1H, d, J=8Hz), 6.1 (1H, s), 2.65-2.55 (2H, m), 2.55 (3H, s), 2.25 (3H, s), 1.6 (2H, m), 1.35 (2H, m), 0.92 (1H, t, J=7Hz)
IR (KBR) cm⁻¹: 3285, 1646, 1075, 702
Elemental analysis (%): Calc'd for C₁₇H₂₁NO₂: C, 75.25; H, 7.80; N, 5.16. Found: C, 75.13; H, 7.87; N, 5.13

### Referential Example 7

### 3'-Sec-butyl-2,5-dimethylfuran-3-carboxanilide

Yield: 38.1%
m.p.: 80.0-81.0°C
¹H NMR (CDCl₃) δ ppm:
7.4 (1H, m), 7.38 (1H, m), 7.25 (1H, b), 7.22 (1H, t, J=8Hz), 6.95 (1H, d, J=8Hz), 6.1 (1H, s), 2.65-2.5 (1H, m), 2.55 (3H, s), 2.25 (3H, s), 1.68-1.5 (2H, m), 1.25 (3H, d, J=7Hz), 0.85 (3H, t, J=7Hz)
IR (KBr) cm⁻¹: 3255, 1647, 1078, 791
Elemental analysis (%): Calc'd for C₁₇H₂₁NO₂: C, 75.25; H, 7.80; N, 5.16. Found: C, 75.19; H, 7.68; N, 5.14

### Referential Example 8

### 3'-Pentyl-2,5-dimethylfuran-3-carboxanilide

Yield: 18.3%
m.p.: 97.0-97.5°C
¹H NMR (CDCl₃) δ ppm:
7.45 (1H, m), 7.35 (1H, m), 7.28 (1H, b), 7.25 (1H, t, J=8Hz), 6.95 (1H, d, J=8Hz), 6.1 (1H, s), 2.65-2.5 (2H, m), 2.55 (3H, s), 2.25 (3H, s), 1.7-1.5 (2H, m), 1.4-1.2 (4H, m), 0.88 (3H, t, J=7Hz)
IR (KBr) cm⁻¹: 3304, 1644, 1077, 710
Elemental analysis (%): Calc'd for C₁₈H₂₃NO₂: C, 75.76; H, 8.12; N, 4.91. Found: C, 75.77; H, 8.18; N, 5.06

The compound having the general formula (I) mentioned above and the composition containing the compound (I) as the active ingredient, with which the present invention are concerned, can be employed by mixing with carriers or, if necessary, with any other additives, followed by the preparation of formulations usually employed such as oil solution, emulsifiable concentrate, solubilizer, paste, wettable powder, flowable, dry flowable, spray and paint, and then the formulation can be used according to any known method for wood preservative treatment. As the additives which are employed suitably to improve the property of the formulation and to strengthen the wood preserving effect, there may be mentioned cationic, anionic and non-ionic surfactants, various high polymer compounds such as methylcellulose and vinyl acetate resin and water-repellents such as silicon oil and paraffin. It is needless to say that combined use may be possible with other wood preservatives, fungicides and bacteriocides including organic iodine compounds such as Sanplas™, IF-1000™ and Troysan™, azole compounds such as Propiconazole™ and Tebuconazole™, Thiabendazole™, Dichlofluanid™ and quaternary ammonium salt compounds; with insecticides including pyrethroids such as Permethrin™, Etofenprox™, Cypermethrin™, Silaneophan™, Tralomethrin™, organic phosphor compounds such as Chlorpyrifos™, Phoxim™ and Propetamphos™, and Imidacroprid™; and with potentiators such as bis-(2,3,3,3-tetrachlorpropyl) ether. An increased effect can be expected by combined use in this manner. In a real case of application, though the content of the compound of the present invention can be changed within a wide range depending on the formulation or on the object, it may usually be suitable to use from 0.1 to 95 weight percent, preferably from 0.2 to 60 weight percent. These formulations are employable in usual methods for wood treatment: for example, coating, dispersal, dipping treatment, mixing, impregnation, or mixing treatment with an adhesive.

Several formulation examples of the compound of the present invention will be shown below, in which it is needless to say that the combination ratio and the kind of additives can be changed widely (in the descriptions below, part means weight part in all cases).

### Test examples of wood preservation

The effectiveness of the wood preservative of the present invention will be explained concretely by the following examples.
(1) According to the test method for wood preservation described in the Japan Industrial Standards [JIS A-9201 (1991)], each of the test compounds was dissolved to a defined concentration in methanol. The solution was impregnated under a reduced pressure into a Sugi (Japanese cedar) sapwood (2 x 2 x 1) cm and then air-dried. Weathering test was repeated 10 times in which one cycle of the treatment was stirring in water for 8 hours, and then heating for 16 hours at 60°C. The test material was placed on the flora of Serpula lacrymans which had been previously grown on a quartz sand medium (malt extract 2%, glucose 1%, peptone 0.3% and yeast 0.2%), and forcedly decayed at 20°C for 12 weeks. From the difference between the dry weight of the test material before the test and that after test, the degree of decrease in weight was obtained. Table 2 shows the results. The test was carried out by use of 9 samples for each condition, and the values shown in Table 2 are the mean values calculated from 9 samples.

**Table 2**

| Sample drugs | Impregnant concentration (%) | Mean weight decrease by decay (%) |
|---|---|---|
| Compound of Example 20 | 0.01 | 0 |
| | 0.005 | 0.1 |
| | | |
| Compound of Example 21 | 0.01 | 0 |
| | 0.005 | 0 |
| | | |
| Control compound 1 | 0.01 | 9.7 |
| | 0.005 | 18.6 |
| | | |
| Without treatment | | 18.4 |
| Control compound 1: 4-Chlorophenyl-3-iodopropargylformal Product of Nagase Co., Ltd.: IF-1000 | | |

From the data shown above, the compound having the general formula (I) prevented decay of the wood samples due to wood-rotting fungi to a significant extent .
(2) Each of 0.1 w/v % methanol solutions of the compound of the present invention and the control drug was impregnated into the test material [a Sugi (Japanese cedar) sapwood, 2 x 2 x 0.5 cm] under a reduced pressure and then air-dried. Weathering test was repeated twice in which one cycle of the treatment was washing (about 2 liter per minute supply) with water for 5 hours, and then heating for 19 hours at 60°C. After dry air sterilization, the test samples were prepared.

The test materials were placed on the flora of Coriolus versicolor which is a lignin-decomposing fungus, and of Tyromyces palustris which is a cellulose-decomposing fungus, and both of which are designated fungal species for assay of wood preservating effect. Both fungi had been previously grown on an agar medium (malt extract 2%, glucose 1% and peptone 0.5%). After the wood samples were forcedly deteriorated at 26°C for 3 weeks, the effectiveness was determined from the degree of hyphal growth on the test material and the presence or absence of lowered maximum crushing strength. Table 3 shows the result.

The wood preventive efficacy was judged by the following criteria.
+: No hyphal growth was observed on the test material, and no difference was found in the maximum crushing strength from the healthy wood sample.
+: A little hyphal growth was observed on the test material, or a little decrease was found in the maximum crushing strength.
-: Hyphal growth was observed on the test material, or clear decrease was found in the maximum crushing strength.

**Table 3**

| Test drug | Coriolus versicolor | Tyromyces palustris |
|---|---|---|
| Example 1 (not part of | + | - |
| the present invention) | | |
| Example 6 | + | + |
| Example 7 | + | - |
| Example 10 | + | - |
| Example 11 | + | + |
| Example 12 | + | + |
| Example 13 | + | + |
| Example 14 | + | + |
| Example 15 | + | - |
| Referential Example 3 | + | + |
| Referential Example 4 | + | + |
| Referential Example 5 | + | + |
| Referential Example 6 | + | + |
| Referential Example 7 | + | + |
| Referential Example 8 | + | + |
| Control compound 2 | + | + |
| Without treatment | - | - |
| Control compound 2: 3-Bromo-2,3-diiodo-2-propenylethylcarbonate Product of Sankyo Co., Ltd.: Sanplas | | |

When the composition of the present invention is desired to be employed, the combination ratio may be suitably selected depending on the kind of wood and the kind of wood material to be treated with the wood preservative, or the means for treatment (for example, coating, dipping, dispersal, impregnation, mixing and mixing with an adhesive). Usually, the combination ratio of dimethylfurancarboxyanilide and any other wood preservative may be from 240:1 to 1:35, preferably from 30:1 to 1:10, and more preferably from 5:1 to 1:5.

The content of the composition of the present invention may be changed within a wide range depending on the formulation. In general, the content may be from 0.1 to 95%, preferably from 0.2 to 60%, in the formulation.

## Claims

1. Dimethylfurancarboxyanilide derivatives represented by the general formula (I): 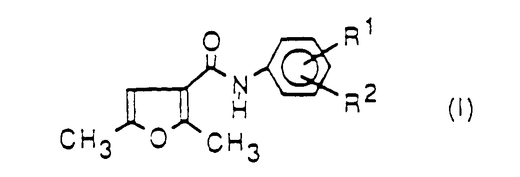 wherein: R¹ represents:
a 3-alkoxycarbonyl group having from 1 to 3 carbon atoms in the alkoxy moiety;
a 3-alkoxymethyl group having from 1 to 3 carbon atoms in the alkoxy moiety;
a cycloalkylcarbonylamino group having from 4 to 6 carbon atoms in the cycloalkyl moiety;
a benzyl group which may be substituted by a methoxy group;
a benzoyl group; or
an alkoxycarbonylalkenyl group having from 1 to 3 carbon atoms in the alkoxy moiety and having from 2 to 3 carbon atoms in the alkenyl moiety; and
R² represents a hydrogen atom.

2. A dimethylfurancarboxyanilide derivative of general formula (I) according to Claim 1 wherein said derivative is 3'-benzyl-2,5-dimethylfuran-3-carboxyanilide.

3. A wood preservative composition comprising a carrier in combination with an amount effective in preserving wood of a dimethylfurancarboxyanilide derivative of general formula (I') as defined below as the active ingredient: 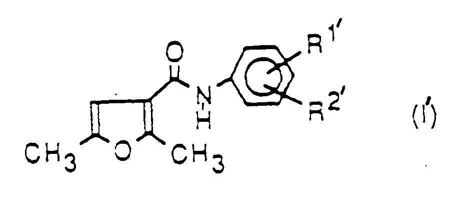 wherein:
R^{1'} represents:
a 3-alkyl group having from 2 to 6 carbon atoms;
a 3-alkoxycarbonyl group having from 1 to 3 carbon atoms in the alkoxy moiety;
a 3-alkoxymethyl group having from 1 to 3 carbon atoms in the alkoxy moiety;
a cycloalkylcarbonylamino group having from 4 to 6 carbon atoms in the cycloalkyl moiety;
a benzyl group which may be substituted by a methoxy group;
a benzoyl group; or
an alkoxycarbonylalkenyl group having from 1 to 3 carbon atoms in the alkoxy moiety and having from 2 to 3 carbon atoms in the alkenyl moiety; and
R^{2'} represents a hydrogen atom.

4. A wood preservative composition according to Claim 3 wherein said dimethylfurancarboxyanilide derivative is 3'-isopropyl-2,5-dimethylfuran-3-carboxyanilide.

5. A method of preserving wood comprising applying to the wood a composition comprising an amount effective in the treatment of wood of a dimethylfurancarboxyanilide derivative of general formula (I') as defined in Claim 3.

6. A method of preserving wood according to Claim 5 wherein said dimethylfurancarboxyanilide derivative is 3'-isopropyl-2,5-dimethylfuran-3-carboxyanilide.

7. A wood preservative composition wherein at least one dimethylfurancarboxyanilide derivative of general formula (I') as defined in Claim 3 is combined with at least one compound selected from 3-bromo-2,3-diiodo-2-propenylethylcarbamate, 3-iodo-2-propynylbutylcarbamate and 4-chlorophenyl-3-iodopropargylformal.

## Patentansprüche

1. Dimethylfurancarboxyanilidderivate, die durch die allgemeine Formel (I) dargestellt sind: 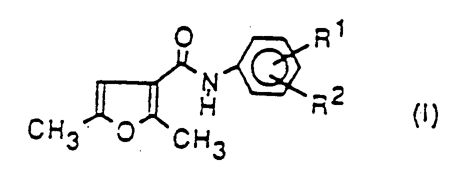 worin:
R¹ eine 3-Alkoxycarbonylgruppe mit 1 bis 3 Kohlenstoffatomen in dem Alkoxyrest, eine 3-Alkoxymethylgruppe mit 1 bis 3 Kohlenstoffatomen in dem Alkoxyrest, eine Cycloalkylcarbonylaminogruppe mit 4 bis 6 Kohlenstoffatomen in dem Cycloalkylrest, eine Benzylgruppe, die mit einer Methoxygruppe substituiert sein kann, eine Benzoylgruppe, oder eine Alkoxycarbonylalkenylgruppe mit 1 bis 3 Kohlenstoffatomen in dem Alkoxyrest und 2 oder 3 Kohlenstoffatomen in dem Alkenylrest darstellt, und
R² ein Wasserstoffatom darstellt.

2. Dimethylfurancarboxyanilidderivat der allgemeinen Formel (I) nach Anspruch 1, worin das Derivat 3'-Benzyl-2,5-dimethylfuran-3-carboxyanilid ist.

3. Holzschutzmittelzusammensetzung, welche einen Träger in Kombination mit einer Menge eines Dimethylfurancarboxyanilidderivats der nachstehend definierten allgemeinen Formel (I'), die wirksam ist, um Holz zu schützen, als aktiven Bestandteil enthält: 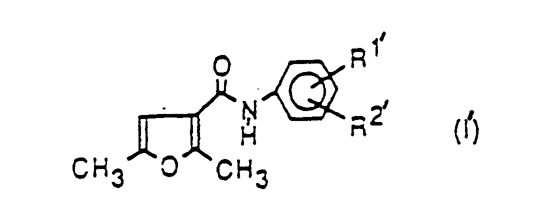 worin:
R^{1'} eine 3-Alkylgruppe mit 2 bis 6 Kohlenstoffatomen, eine 3-Alkoxycarbonylgruppe mit 1 bis 3 Kohlenstoffatomen in dem Alkoxyrest, eine 3-Alkoxymethylgruppe mit 1 bis 3 Kohlenstoffatomen in dem Alkoxyrest, eine Cycloalkylcarbonylaminogruppe mit 4 bis 6 Kohlenstoffatomen in dem Cycloalkylrest, eine Benzylgruppe, die mit einer Methoxygruppe substituiert sein kann, eine Benzoylgruppe, oder eine Alkoxycarbonylalkenylgruppe mit 1 bis 3 Kohlenstoffatomen in dem Alkoxyrest und mit 2 oder 3 Kohlenstoffatomen in dem Alkenylrest darstellt, und
R²' ein Wasserstoffatom darstellt.

4. Holzschutzmittelzusammensetzung nach Anspruch 3, worin das Dimethylfurancarboxyanilidderivat 3'-Isopropyl-2,5-dimethylfuran-3-carboxyanilid ist.

5. Verfahren zum Schützen von Holz, welches das Auftragen einer Zusammensetzung auf Holz umfaßt, die eine Menge eines Dimethylfurancarboxyanilidderivats der in Anspruch 3 definierten allgemeinen Formel (I'), die bei der Behandlung von Holz effektiv ist, enthält.

6. Verfahren zum Schützen von Holz nach Anspruch 5, worin das Dimethylfurancarboxyanilidderivat 3'-Isopropyl-2,5-dimethylfuran-3-carboxyanilid ist.

7. Holzschutzmittelzusammensetzung, worin mindestens ein Dimethylfurancarboxyanilidderivat der in Anspruch 3 definierten allgemeinen Formel (I') mit mindestens einer Verbindung kombiniert ist, die unter 3-Brom-2,3-diiod-2-propenylethylcarbamat, 3-Iod-2-propinylbutylcarbamat und 4-Chlorphenyl-3-iodpropargylformal ausgewählt ist.

## Revendications

1. Dérivés d'anilidocarboxydiméthylfuranne représentés par la formule générale (I) : 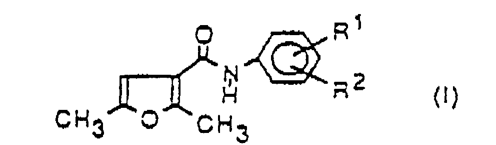 dans laquelle :
R¹ représente :
un groupe 3-alcoxycarbonyle ayant 1 à 3 atomes de carbone dans le fragment alcoxy ;
un groupe 3-alcoxyméthyle ayant 1 à 3 atomes de carbone dans le fragment alcoxy ;
un groupe cycloalkylcarbonylamino ayant 4 à 6 atomes de carbone dans le fragment cycloalkyle ;
un groupe benzyle qui peut être substitué par un groupe méthoxy ;
un groupe benzoyle ; ou
un groupe alcoxycarbonylalcényle ayant 1 à 3 atomes de carbone dans le fragment alcoxy et ayant 2 ou 3 atomes de carbone dans le fragment alcényle ; et
R² représente un atome d'hydrogène.

2. Dérivé d'anilidocarboxydiméthylfuranne de formule générale (I) selon la revendication 1, ledit dérivé étant le 3'-benzyl-2,5-diméthylfuranne-3-carboxyanilide.

3. Composition de protection du bois comprenant un véhicule en association avec, en quantité efficace pour assurer la protection du bois, un dérivé d'anilidocarboxydiméthylfuranne de formule générale (I') telle que définie ci-dessous comme ingrédient actif : 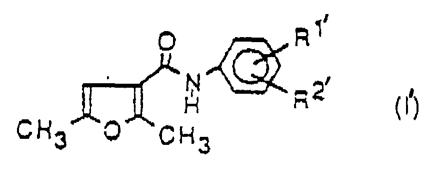 dans laquelle :
R^{1'} représente :
un groupe 3-alkyle ayant 2 à 6 atomes de carbone ;
un groupe 3-alcoxycarbonyle ayant 1 à 3 atomes de carbone dans le fragment alcoxy ;
un groupe 3-alcoxyméthyle ayant 1 à 3 atomes de carbone dans le fragment alcoxy ;
un groupe cycloalkylcarbonylamino ayant 4 à 6 atomes de carbone dans le fragment cycloalkyle ;
un groupe benzyle qui peut être substitué par un groupe méthoxy ;
un groupe benzoyle ; ou
un groupe alcoxycarbonylalcényle ayant 1 à 3 atomes de carbone dans le fragment alcoxy et ayant 2 ou 3 atomes de carbone dans le fragment alcényle ; et
R^{2'} représente un atome d'hydrogène.

4. Composition de protection du bois selon la revendication 3, dans laquelle ledit dérivé d'anilidocarboxydiméthylfuranne est le 3'-isopropyl-2,5-diméthylfuranne-3-carboxanilide.

5. Procédé de protection du bois, comprenant une étape consistant à appliquer sur le bois une composition comprenant une quantité, efficace pour le traitement du bois, d'un dérivé d'anilidocarboxydiméthylfuranne de formule générale (I'), telle que définie dans la revendication 3.

6. Procédé de protection du bois selon la revendication 5, dans lequel ledit dérivé d'anilidocarboxydiméthylfuranne est le 3'-isopropyl-2,5-diméthylfuranne-3-carboxyanilide.

7. Composition de protection du bois dans laquelle au moins un dérivé d'anilidocarboxydiméthylfuranne de formule générale (I') telle que définie dans la revendication 3, est associé à au moins un composé choisi parmi l'éthylcarbamate de 3-bromo-2,3-diiodo-2-propényle, le butylcarbamate de 3-iodo-2-propynyle et le 4-chlorophényl-3-iodopropargylformal.
